# EUROPEAN PATENT APPLICATION

(11) **EP 4 678 100 A1**
(43) Date of publication of application: **14.01.2026**
(21) Application number: 25187843.5
(22) Date of filing: 07.07.2025
(51) Int. Cl.: A61B 5/12, A61B 5/00

(54) **SYNCHRONIZED EVOKED RESPONSE MEASUREMENTS IN A BILATERAL HEARING SYSTEM**

(30) Priority: 08.07.2024 US 202463668660 P
(71) Applicant: Advanced Bionics, LLC, Valencia CA 91355 (US)
(72) Inventor: Chen, Chen, Valencia (US); Koka, Kanthaiah, Valencia (US); Spahr, Anthony J., Newhall (US); Patnala, Anil K., Stevenson Ranch (US); Salek, Kurt, Santa Clarita (US)
(74) Representative: Schwan Schorer & Partner mbB

(57) **Abstract**

An illustrative system is configured to perform a process comprising: directing stimulation to be applied to a recipient of a bilateral hearing system that includes a first cochlear implant associated with a first ear of the recipient and a second cochlear implant associated with a second ear of the recipient; and causing the first cochlear implant and the second cochlear implant to simultaneously record, during an acquisition time window, one or more evoked responses within the recipient that are elicited by the stimulation.

## Description

### RELATED APPLICATIONS

The present application claims priority to U.S. Provisional Patent Application No. 63/668,660, filed July 8, 2024, the contents of which is hereby incorporated by reference in its entirety.

### BACKGROUND INFORMATION

Cochlear implant systems are used to provide, restore, and/or improve the sense of hearing to recipients with severe or profound hearing loss. Conventional cochlear implant systems include various components configured to be implanted within a recipient (e.g., an electronics package, an antenna, and an electrode lead) and various components configured to be located external to the recipient (e.g., a sound processor, a battery, and a microphone).

An electrode lead of a cochlear implant system may include an electrode array comprised of metal electrode contacts (e.g., platinum, titanium, etc.) insulated by the medical grade silicone. The metal electrode contacts are configured to be inserted within a cochlea of a recipient and are typically used to apply electrical stimulation to one or more intracochlear locations.

When stimulation is applied to the recipient of a cochlear implant system, the metal electrode contacts on the electrode array may also be used to measure an evoked response, such as an electrocochleographic ("ECochG") potential (e.g., a cochlear microphonic potential, a compound action potential such as an auditory nerve response, a summating potential, etc.), a brainstem response (e.g., central auditory potentials of a central auditory pathway of a recipient), a stapedius reflex, and/or any other type of neural or physiological response that may occur in response to the stimulation.

Evoked responses often occur in both hemispheres of the brain. Heretofore, only unilateral evoked response measurements (i.e., measurements from only one side) have been performed. This results in lost information, increased measurement times, and the inability to compare measurements taken simultaneously at different locations.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
Fig. 1 illustrates an illustrative cochlear implant system.
Fig. 2 shows an illustrative configuration of the cochlear implant system of Fig. 1.
Fig. 3 shows another illustrative configuration of the cochlear implant system of Fig. 1.
Fig. 4 illustrates an example bilateral hearing system.
Fig. 5 shows an illustrative evoked response detection system.
Fig. 6 shows an illustrative method.
Fig. 7 shows an illustrative method.
Fig. 8 shows an example configuration of a bilateral hearing system.
Fig. 9 shows an example configuration of a bilateral hearing system.
Fig. 10 shows an illustrative computing device.

### DETAILED DESCRIPTION

Systems and methods for performing synchronized evoked response measurements in a bilateral hearing system are described herein. An illustrative system is configured to perform a process that includes 1) directing stimulation to be applied to a recipient of a bilateral hearing system that includes a first cochlear implant associated with a first ear of the recipient and a second cochlear implant associated with a second ear of the recipient, and 2) causing the first cochlear implant and the second cochlear implant to simultaneously record, during an acquisition time window, one or more evoked responses within the recipient that are elicited by the stimulation. The process may further include 1) receiving, from the first cochlear implant by way of a first back telemetry channel, first evoked response data representative of the one or more evoked responses recorded by the first cochlear implant, 2) receiving, from the second cochlear implant by way of a second back telemetry channel, second evoked response data representative of the one or more evoked responses recorded by the second cochlear implant, and 3) associating the first evoked response data and the second evoked response data with metadata representative of one or more attributes of the stimulation.

As described herein, by facilitating simultaneous recording of evoked response signals by both cochlear implants included in a bilateral hearing system, the systems and methods described herein may be configured to allow a computing device to perform various operations based the simultaneously recorded evoked response signals. For example, the computing device may compare the signals to determine how a particular stimulus relatively affects different parts of the brain, perform one or more diagnostic operations based on the simultaneous records, adjust one or more operating parameters associated with the bilateral hearing system (e.g., by adjusting stimulation parameters that define the type of stimulation that is to be provided by the bilateral cochlear implants to represent sound to the recipient, etc.).

Various embodiments will now be described in more detail with reference to the figures. The disclosed systems and methods may provide one or more of the benefits mentioned above and/or various additional and/or alternative benefits that will be made apparent herein.

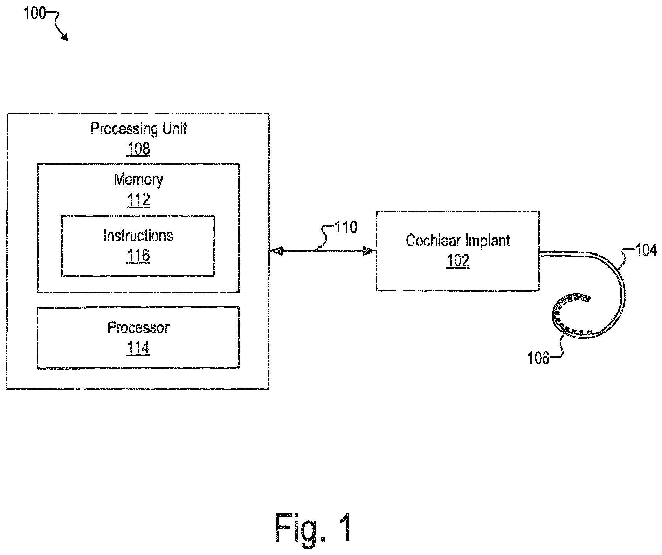

Fig. 1 illustrates an illustrative cochlear implant system 100 configured to be used by a recipient. As shown, cochlear implant system 100 includes a cochlear implant 102, an electrode lead 104 physically coupled to cochlear implant 102 and having an array of electrodes 106, and a processing unit 108 configured to be communicatively coupled to cochlear implant 102 by way of a communication link 110.

The cochlear implant system 100 shown in Fig. 1 is unilateral (i.e., associated with only one ear of the recipient). Alternatively, as described herein, a bilateral configuration of cochlear implant system 100 may include separate cochlear implants and electrode leads for each ear of the recipient. In the bilateral configuration, processing unit 108 may be implemented by a single processing unit configured to interface with both cochlear implants or by two separate processing units each configured to interface with a different one of the cochlear implants.

Cochlear implant 102 may be implemented by any suitable type of implantable stimulator. For example, cochlear implant 102 may be implemented by an implantable cochlear stimulator. Additionally or alternatively, cochlear implant 102 may be implemented by a brainstem implant and/or any other type of device that may be implanted within the recipient and configured to apply electrical stimulation to one or more stimulation sites located along an auditory pathway of the recipient.

In some examples, cochlear implant 102 may be configured to generate electrical stimulation representative of an audio signal processed by processing unit 108 in accordance with one or more stimulation parameters transmitted to cochlear implant 102 by processing unit 108. Cochlear implant 102 may be further configured to apply the electrical stimulation to one or more stimulation sites (e.g., one or more intracochlear locations) within the recipient by way of one or more electrodes 106 on electrode lead 104. In some examples, cochlear implant 102 may include a plurality of independent current sources each associated with a channel defined by one or more of electrodes 106. In this manner, different stimulation current levels may be applied to multiple stimulation sites simultaneously by way of multiple electrodes 106.

Cochlear implant 102 may additionally or alternatively be configured to generate, store, and/or transmit data. For example, cochlear implant may use one or more electrodes 106 to record one or more signals (e.g., one or more voltages, impedances, evoked responses within the recipient, and/or other measurements) and transmit, by way of communication link 110, data representative of the one or more signals to processing unit 108. In some examples, this data is referred to as back telemetry data.

Electrode lead 104 may be implemented in any suitable manner. For example, a distal portion of electrode lead 104 may be pre-curved such that electrode lead 104 conforms with the helical shape of the cochlea after being implanted. Electrode lead 104 may alternatively be naturally straight or of any other suitable configuration.

In some examples, electrode lead 104 includes a plurality of wires (e.g., within an outer sheath) that conductively couple electrodes 106 to one or more current sources within cochlear implant 102. For example, if there are n electrodes 106 on electrode lead 104 and n current sources within cochlear implant 102, there may be n separate wires within electrode lead 104 that are configured to conductively connect each electrode 106 to a different one of the n current sources. Illustrative values for n are 8, 12, 16, or any other suitable number.

Electrodes 106 are located on at least a distal portion of electrode lead 104. In this configuration, after the distal portion of electrode lead 104 is inserted into the cochlea, electrical stimulation may be applied by way of one or more of electrodes 106 to one or more intracochlear locations. One or more other electrodes (e.g., including a ground electrode, not explicitly shown) may also be disposed on other parts of electrode lead 104 (e.g., on a proximal portion of electrode lead 104) to, for example, provide a current return path for stimulation current applied by electrodes 106 and to remain external to the cochlea after the distal portion of electrode lead 104 is inserted into the cochlea. Additionally or alternatively, a housing of cochlear implant 102 may serve as a ground electrode for stimulation current applied by electrodes 106.

Processing unit 108 may be configured to interface with (e.g., control and/or receive data from) cochlear implant 102. For example, processing unit 108 may transmit commands (e.g., stimulation parameters and/or other types of operating parameters in the form of data words included in a forward telemetry sequence) to cochlear implant 102 by way of communication link 110. Processing unit 108 may additionally or alternatively provide operating power to cochlear implant 102 by transmitting one or more power signals to cochlear implant 102 by way of communication link 110. Processing unit 108 may additionally or alternatively receive data from cochlear implant 102 by way of communication link 110. Communication link 110 may be implemented by any suitable number of wired and/or wireless bidirectional and/or unidirectional links, such as an inductive link.

As shown, processing unit 108 includes a memory 112 and a processor 114 configured to be selectively and communicatively coupled to one another. In some examples, memory 112 and processor 114 may be distributed between multiple devices and/or multiple locations as may serve a particular implementation.

Memory 112 may be implemented by any suitable non-transitory computer-readable medium and/or non-transitory processor-readable medium, such as any combination of non-volatile storage media and/or volatile storage media. Illustrative non-volatile storage media include, but are not limited to, read-only memory, flash memory, a solid-state drive, a magnetic storage device (e.g., a hard drive), ferroelectric random-access memory ("RAM"), and an optical disc. Illustrative volatile storage media include, but are not limited to, RAM (e.g., dynamic RAM).

Memory 112 may maintain (e.g., store) executable data used by processor 114 to perform one or more of the operations described herein. For example, memory 112 may store instructions 116 that may be executed by processor 114 to perform any of the operations described herein. Instructions 116 may be implemented by any suitable application, program (e.g., sound processing program), software, code, and/or other executable data instance. Memory 112 may also maintain any data received, generated, managed, used, and/or transmitted by processor 114.

Processor 114 may be configured to perform (e.g., execute instructions 116 stored in memory 112 to perform) various operations with respect to cochlear implant 102.

To illustrate, processor 114 may be configured to control an operation of cochlear implant 102. For example, processor 114 may receive an audio signal (e.g., by way of a microphone communicatively coupled to processing unit 108, a wireless interface (e.g., a Bluetooth interface), and/or a wired interface (e.g., an auxiliary input port)). Processor 114 may process the audio signal in accordance with a sound processing program (e.g., a sound processing program stored in memory 112) to generate appropriate stimulation parameters. Processor 114 may then transmit the stimulation parameters to cochlear implant 102 to direct cochlear implant 102 to apply electrical stimulation representative of the audio signal to the recipient.

In some implementations, processor 114 may also be configured to apply acoustic stimulation to the recipient. For example, a receiver (also referred to as a loudspeaker) may be optionally coupled to processing unit 108. In this configuration, processor 114 may deliver acoustic stimulation to the recipient by way of the receiver. The acoustic stimulation may be representative of an audio signal (e.g., an amplified version of the audio signal), configured to elicit an evoked response within the recipient, and/or otherwise configured. In configurations in which processor 114 is configured to both deliver acoustic stimulation to the recipient (e.g., at a left ear) and direct cochlear implant 102 to apply electrical stimulation to the recipient (e.g., at a right ear), cochlear implant system 100 may be referred to as a bimodal hearing system and/or any other suitable term.

Processor 114 may be additionally or alternatively configured to receive and process data generated by cochlear implant 102. For example, processor 114 may receive data representative of a signal recorded by cochlear implant 102 using one or more electrodes 106 and, based on the data, adjust one or more operating parameters of processing unit 108. Additionally or alternatively, processor 114 may use the data to perform one or more diagnostic operations with respect to cochlear implant 102 and/or the recipient.

Other operations may be performed by processor 114 as may serve a particular implementation. In the description provided herein, any references to operations performed by processing unit 108 and/or any implementation thereof may be understood to be performed by processor 114 based on instructions 116 stored in memory 112.

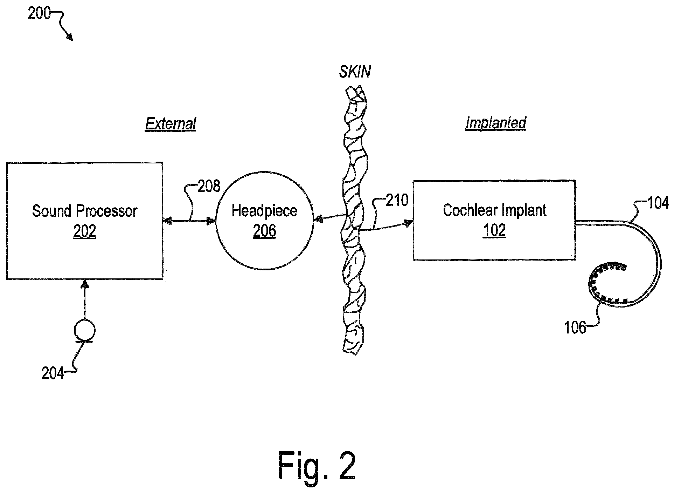

Processing unit 108 may be implemented by one or more devices configured to interface with cochlear implant 102. To illustrate, Fig. 2 shows an illustrative configuration 200 of cochlear implant system 100 in which processing unit 108 is implemented by a sound processor 202 configured to be located external to the recipient. In configuration 200, sound processor 202 is communicatively coupled to a microphone 204 and to a headpiece 206 that are both configured to be located external to the recipient.

Sound processor 202 may be implemented by any suitable device that may be worn or carried by the recipient. For example, sound processor 202 may be implemented by a behind-the-ear ("BTE") unit configured to be worn behind and/or on top of an ear of the recipient. Additionally or alternatively, sound processor 202 may be implemented by an off-the-ear unit (also referred to as a body worn device) configured to be worn or carried by the recipient away from the ear. Additionally or alternatively, at least a portion of sound processor 202 is implemented by circuitry within headpiece 206.

Microphone 204 is configured to detect one or more audio signals (e.g., that include speech and/or any other type of sound) in an environment of the recipient. Microphone 204 may be implemented in any suitable manner. For example, microphone 204 may be implemented by a microphone that is configured to be placed within the concha of the ear near the entrance to the ear canal, such as a T-MIC^{™} microphone from Advanced Bionics. Such a microphone may be held within the concha of the ear near the entrance of the ear canal during normal operation by a boom or stalk that is attached to an ear hook configured to be selectively attached to sound processor 202. Additionally or alternatively, microphone 204 may be implemented by one or more microphones in or on headpiece 206, one or more microphones in or on a housing of sound processor 202, one or more beam-forming microphones, and/or any other suitable microphone as may serve a particular implementation.

Headpiece 206 may be selectively and communicatively coupled to sound processor 202 by way of a communication link 208 (e.g., a cable or any other suitable wired or wireless communication link), which may be implemented in any suitable manner. Headpiece 206 may include an external antenna (e.g., a coil and/or one or more wireless communication components) configured to facilitate selective wireless coupling of sound processor 202 to cochlear implant 102. Headpiece 206 may additionally or alternatively be used to selectively and wirelessly couple any other external device to cochlear implant 102. To this end, headpiece 206 may be configured to be affixed to the recipient's head and positioned such that the external antenna housed within headpiece 206 is communicatively coupled to a corresponding implantable antenna (which may also be implemented by a coil and/or one or more wireless communication components) included within or otherwise connected to cochlear implant 102. In this manner, stimulation parameters and/or power signals may be wirelessly and transcutaneously transmitted between sound processor 202 and cochlear implant 102 by way of a wireless communication link 210.

In configuration 200, sound processor 202 may receive an audio signal detected by microphone 204 by receiving a signal (e.g., an electrical signal) representative of the audio signal from microphone 204. Sound processor 202 may additionally or alternatively receive the audio signal by way of any other suitable interface as described herein. Sound processor 202 may process the audio signal in any of the ways described herein and transmit, by way of headpiece 206, stimulation parameters to cochlear implant 102 to direct cochlear implant 102 to apply electrical stimulation representative of the audio signal to the recipient.

In an alternative configuration, sound processor 202 may be implanted within the recipient instead of being located external to the recipient. In this alternative configuration, which may be referred to as a fully implantable configuration of cochlear implant system 100, sound processor 202 and cochlear implant 102 may be combined into a single device or implemented as separate devices configured to communicate one with another by way of a wired and/or wireless communication link. In a fully implantable implementation of cochlear implant system 100, headpiece 206 may not be included and microphone 204 may be implemented by one or more microphones implanted within the recipient, located within an ear canal of the recipient, and/or external to the recipient.

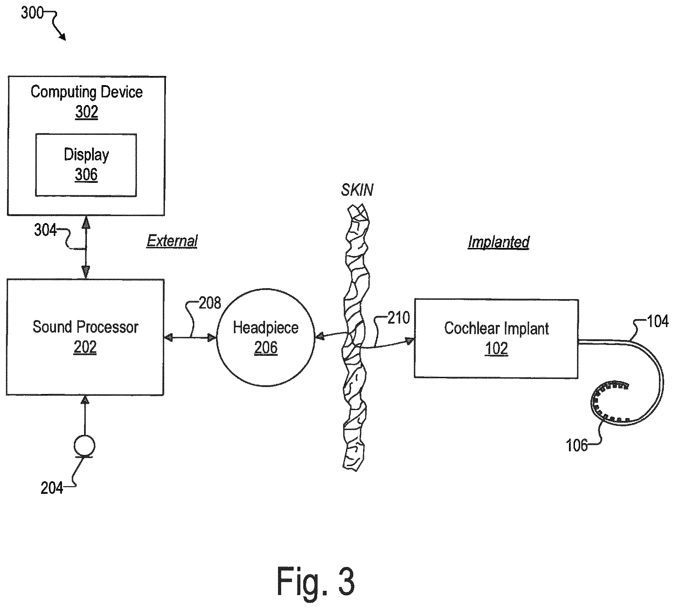

Fig. 3 shows an illustrative configuration 300 of cochlear implant system 100 in which processing unit 108 is implemented by a combination of sound processor 202 and a computing device 302 configured to communicatively couple to sound processor 202 by way of a communication link 304, which may be implemented by any suitable wired or wireless communication link.

Computing device 302 may be implemented by any suitable combination of hardware and software. To illustrate, computing device 302 may be implemented by a mobile device (e.g., a mobile phone, a laptop, a tablet computer, etc.), a desktop computer, and/or any other suitable computing device as may serve a particular implementation. As an example, computing device 302 may be implemented by a mobile device configured to execute an application (e.g., a "mobile app") that may be used by a user (e.g., the recipient, a clinician, and/or any other user) to control one or more settings of sound processor 202 and/or cochlear implant 102 and/or perform one or more operations (e.g., diagnostic operations) with respect to data generated by sound processor 202 and/or cochlear implant 102.

In some examples, computing device 302 may be configured to control an operation of cochlear implant 102 by transmitting one or more commands to cochlear implant 102 by way of sound processor 202. Likewise, computing device 302 may be configured to receive data generated by cochlear implant 102 by way of sound processor 202. Alternatively, computing device 302 may interface with (e.g., control and/or receive data from) cochlear implant 102 directly by way of a wireless communication link between computing device 302 and cochlear implant 102. In some implementations in which computing device 302 interfaces directly with cochlear implant 102, sound processor 202 may or may not be included in cochlear implant system 100.

Computing device 302 is shown as having an integrated display 306. Display 306 may be implemented by a display screen, for example, and may be configured to display content generated by computing device 302. Additionally or alternatively, computing device 302 may be communicatively coupled to an external display device (not shown) configured to display the content generated by computing device 302.

In some examples, computing device 302 represents a fitting device configured to be selectively used (e.g., by a clinician) to fit sound processor 202 and/or cochlear implant 102 to the recipient. In these examples, computing device 302 may be configured to execute a fitting program configured to set one or more operating parameters of sound processor 202 and/or cochlear implant 102 to values that are optimized for the recipient. As such, in these examples, computing device 302 may not be considered to be part of cochlear implant system 100. Instead, computing device 302 may be considered to be separate from cochlear implant system 100 such that computing device 302 may be selectively coupled to cochlear implant system 100 when it is desired to fit sound processor 202 and/or cochlear implant 102 to the recipient.

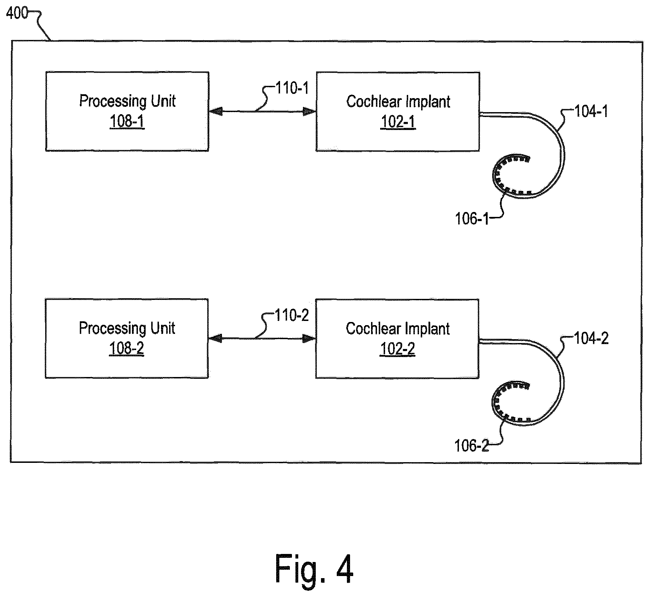

Fig. 4 illustrates an example bilateral hearing system 400. As shown, bilateral hearing system 400 includes a cochlear implant 102-1, an electrode lead 104-1 physically coupled to cochlear implant 102-1 and having an array of electrodes 106-1, and a processing unit 108-1 configured to be communicatively coupled to cochlear implant 102-1 by way of a communication link 110-1. Each of these components is associated with a first ear of a recipient. For example, cochlear implant 102-1 may use electrodes 106-1 to apply electrical stimulation to a cochlea associated with the first ear and, as described herein, record one or more evoked responses within the recipient that are elicited by the electrical stimulation (and/or any other type of stimulation).

Bilateral hearing system 400 further includes a cochlear implant 102-2, an electrode lead 104-2 physically coupled to cochlear implant 102-2 and having an array of electrodes 106-2, and a processing unit 108-2 configured to be communicatively coupled to cochlear implant 102-2 by way of a communication link 110-2. Each of these components is associated with a second ear of a recipient. For example, cochlear implant 102-2 may use electrodes 106-2 to apply electrical stimulation to a cochlea associated with the second ear and, as described herein, record one or more evoked responses within the recipient that are elicited by the electrical stimulation (and/or any other type of stimulation).

Processing units 108-1 and 108-2 may be similar to processing unit 108 described in connection with Fig. 1. For example, each processing unit 108-1 and 108-2 may be implemented by a sound processor (e.g., a BTE unit) and/or a computing device (e.g., a fitting device). While two processing units 108-1 and 108-2 are depicted in Fig. 4, in some alternative embodiments a single processing unit may be communicatively coupled to and configured to control both cochlear implants 102-1 and 102-2.

Electrodes electrically coupled to a cochlear implant (e.g., any of the cochlear implants described herein may be used in any suitable manner to measure an evoked response elicited within a recipient of the cochlear implant by stimulation (e.g., electrical stimulation and/or acoustic stimulation). As used herein, an "evoked response" may include any type of cochlear response and/or neural response. Exemplary evoked responses include, but are not limited to, an electrocochleographic ("ECochG") potential (e.g., a cochlear microphonic potential, a compound action potential such as an auditory nerve response, a summating potential, etc.), a brainstem response (e.g., central auditory potentials of a central auditory pathway of a recipient), a stapedius reflex, and/or any other type of neural or physiological response that may occur within a recipient in response to application of stimulation to the recipient. Evoked responses may originate from neural tissues, hair cell to neural synapses, inner or outer hair cells, and/or other sources.

For example, in bilateral hearing system 400 depicted in Fig. 4, one or more of electrodes 106-1 and one or more of electrodes 106-2 may be used to record an evoked response that occurs in response to electrical stimulation applied by cochlear implant 102-1 and/or cochlear implant 102-2. Because electrodes 106-1 are located in a cochlea associated with a first ear (e.g., the right ear) of the recipient and electrodes 106-2 are located in a cochlea associated with a second ear (e.g., the left ear) of the recipient, evoked responses recorded by electrodes 106-1 may originate in a first hemisphere (e.g., the right hemisphere) of a brain of the recipient and evoked responses recorded by electrodes 106-2 may originate in a second hemisphere (e.g., the left hemisphere) of the brain of the recipient.

Evoked responses such as those described herein may be used in any suitable manner. For example, evoked responses may be used to fit a hearing device (e.g., a cochlear implant, a hearing aid, etc.) to a recipient, to evaluate the recipient's hearing capability, and/or to monitor auditory brain maturation (e.g., pediatric brain development in relation to the effectiveness of a cochlear implant).

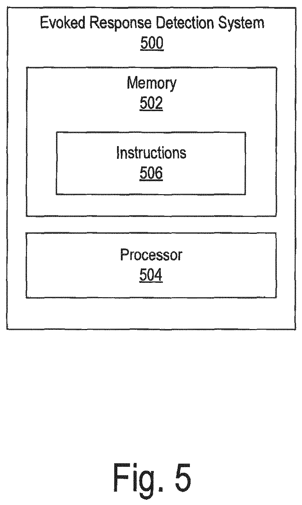

Fig. 5 shows an illustrative evoked response detection system 500 ("system 500") that may be implemented according to principles described herein. As shown, system 500 may include, without limitation, a memory 502 and a processor 504 selectively and communicatively coupled to one another. Memory 502 and processor 504 may each include or be implemented by hardware and/or software components (e.g., processors, memories, communication interfaces, instructions stored in memory for execution by the processors, etc.). In some examples, memory 502 and/or processor 504 may be implemented by any suitable computing device such as described herein. In other examples, memory 502 and/or processor 504 may be distributed between multiple devices and/or multiple locations as may serve a particular implementation. Illustrative implementations of system 500 are described herein.

Memory 502 may maintain (e.g., store) executable data used by processor 504 to perform any of the operations described herein. For example, memory 502 may store instructions 506 that may be executed by processor 504 to perform any of the operations described herein. Instructions 506 may be implemented by any suitable application, software, code, and/or other executable data instance.

Memory 502 may also maintain any data received, generated, managed, used, and/or transmitted by processor 504. Memory 502 may store any other suitable data as may serve a particular implementation. For example, memory 502 may store hearing loss profile data, recipient data (e.g., age at implantation, duration of implantation, etc.), evoked response data, electrode position data, dipole data (e.g., dipole location, orientation, polarity, etc.), setting data, stimulation parameter data, graphical user interface content, and/or any other suitable data.

Processor 504 may be configured to perform (e.g., execute instructions 506 stored in memory 502 to perform) various processing operations associated with obtaining evoked response measurements within a bilateral hearing system (e.g., bilateral hearing system 400).

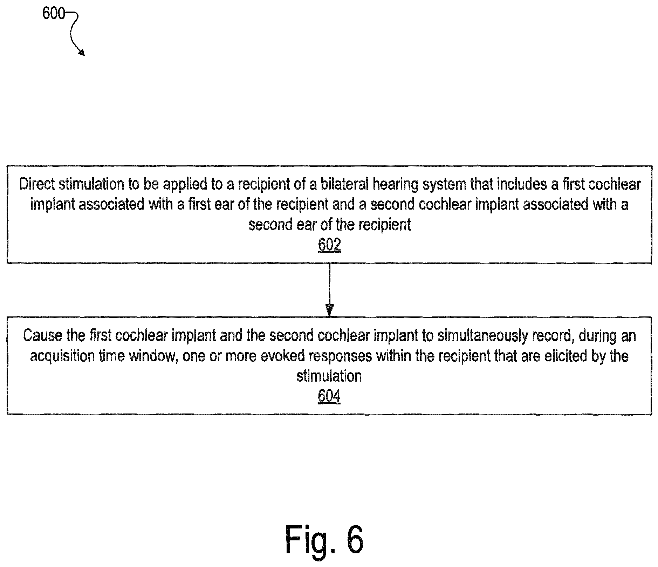

For example, Fig. 6 shows an illustrative method 600 that may be performed by system 500. While Fig. 6 shows illustrative operations according to one embodiment, other embodiments may omit, add to, reorder, and/or modify any of the operations shown in Fig. 6. Moreover, each of the operations depicted in Fig. 6 may be performed in any of the ways described herein.

At operation 602, system 500 may direct stimulation to be applied to a recipient of a bilateral hearing system that includes a first cochlear implant (e.g., cochlear implant 102-1) associated with a first ear of the recipient and a second cochlear implant (e.g., cochlear implant 102-2) associated with a second ear of the recipient.

The stimulation may include electrical stimulation and/or acoustic stimulation. For example, system 500 may direct the first cochlear implant to apply electrical stimulation to the recipient by way of at least one electrode electrically coupled to the first cochlear implant. Additionally or alternatively, system 500 may direct the second cochlear implant to apply additional electrical stimulation to the recipient by way of at least one electrode electrically coupled to the second cochlear implant. Additionally or alternatively, system 500 may apply (or direct another device to apply) acoustic stimulation to the recipient by way of one or more speakers in a vicinity of one or both ears of the recipient. The electrical and/or acoustic stimulation may be applied simultaneously or sequentially as may serve a particular implementation.

At operation 604, system 500 may cause the first cochlear implant and the second cochlear implant to simultaneously record, during an acquisition time window, one or more evoked responses within the recipient that are elicited by the stimulation. As described herein, the cochlear implants may be configured to record the one or more evoked responses using at least one electrode electrically connected thereto.

As used herein, an "acquisition time window" refers to a period of time during which a cochlear implant is in a recording mode in which the cochlear implant uses at least one electrode to record one or more evoked responses. The acquisition time window may have any suitable duration as may serve a particular implementation.

In some examples, the cochlear implant may continuously record evoked response data during the acquisition time window. As used herein, "continuously record" may mean that the cochlear implant is in a recording mode during the entire acquisition time window such that the electrodes are used to record evoked responses any time within the acquisition time window. In certain examples, the "continuously recording" may include minimal gaps as long as the gaps do not shadow/void any evoked response characteristics of interest in a meaningful way. The continuously recording of the data may include measuring a signal between the at least one electrode and an additional electrode electrically coupled to the cochlea implant. For example, one or more of electrodes on an electrode lead may be used as measuring electrodes and a ring electrode on the electrode lead or a ground electrode on the cochlear implant may be used as a return electrode.

System 500 may cause the first cochlear implant and the second cochlear implant to simultaneously record one or more evoked responses during an acquisition time window in any suitable manner. For example, system 500 may transmit a first command to the first cochlear implant and the second cochlear implant that causes the first cochlear implant and the second cochlear implant to start recording the one or more evoked responses at a first point in time that defines a beginning of the acquisition time window. In response to receiving the first command, the cochlear implants may start recording the one or more evoked responses at substantially the same time. Starting to record evoked responses "at the same time" refers to both cochlear implant starting to record evoked responses immediately in response to receiving the first command and/or at a specific point in time as defined by the first command. It will be recognized that slight variances in start times between the two cochlear implants may occur due to any of a number of factors and result in the cochlear implants starting to record the one or more evoked responses at slightly different start times. However, as long as these differences are within a tolerance threshold amount of time, the cochlear implants may be considered to start recording the one or more evoked responses at the same time.

In some examples, system 500 may also transmit a second command to the first cochlear implant and the second cochlear implant that causes the first cochlear implant and the second cochlear implant to stop recording the one or more evoked responses at a second point in time that defines an end of the acquisition time window. Again, slight variances in the stop time between the two cochlear implants may occur due to any of a number of factors and result in the cochlear implants stopping to record the one or more evoked responses at slightly different stop times. However, as long as these differences are within a tolerance threshold amount of time, the cochlear implants may be considered to stop recording the one or more evoked responses at the same time.

The first and second commands may be in any suitable format. For example, the first and second commands may be trigger signals that the cochlear implants are configured to execute immediately in response to receiving the first and second commands. Alternatively, the first and second commands may include instructions to start and stop recording at specific points in time. In some alternative embodiments, only a single command is sent to the first and second cochlear implants, where the single command is configured to cause the cochlear implants to start recording the one or more evoked responses at the same time and to stop recording the one or more evoked responses at the same time.

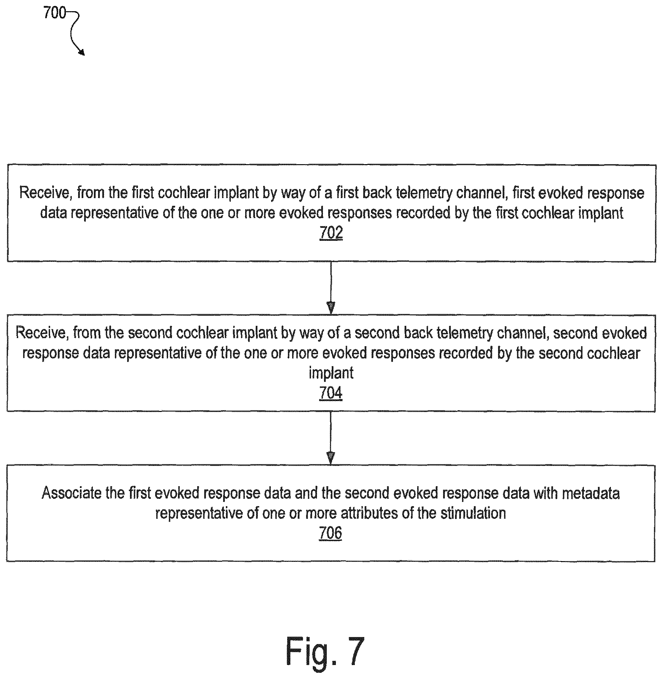

Fig. 7 shows another illustrative method 700 that may be performed by system 500 based on causing the first and second cochlear implants to simultaneously record, during the acquisition time window, the one or more evoked responses. While Fig. 7 shows illustrative operations according to one embodiment, other embodiments may omit, add to, reorder, and/or modify any of the operations shown in Fig. 7. Moreover, each of the operations depicted in Fig. 7 may be performed in any of the ways described herein.

At operation 702, system 500 may receive, from the first cochlear implant by way of a first back telemetry channel (e.g., a back telemetry channel implemented by way of communication link 110-1), first evoked response data representative of the one or more evoked responses recorded by the first cochlear implant. At operation 704, system 500 may receive, from the second cochlear implant by way of a second back telemetry channel (e.g., a back telemetry channel implemented by way of communication link 110-2), second evoked response data representative of the one or more evoked responses recorded by the second cochlear implant.

The first and second cochlear implants may transmit the first and second evoked response data to system 500 in any suitable manner. For example, system 500 may direct the cochlear implants to stream the first and second evoked response data by way of the first and second back telemetry channels to system 500 during the acquisition time window. The evoked response data may be streamed to system 500 by way of the back telemetry channels in any suitable manner. For example, in certain implementations, the evoked response data may be streamed directly from the cochlear implants to system 500 by way of the back telemetry channels. In certain alternative implementations, the evoked response data may be streamed to headpieces of the associated with the cochlear implants and the headpieces may then transmit the evoked response data to system 500 in any suitable manner using any suitable communication protocol.

In certain examples, the cochlear implants may not store the evoked response data in a buffer of the cochlear implants prior to streaming the data to system 500. Rather, the evoked response data may be continuously streamed to system 500 by way of the back telemetry channels in real time or near real time as the evoked response data is recorded by way of the electrodes. By continually recording the evoked response data and simultaneously streaming the evoked response data by way of the back telemetry channels, it is possible to measure evoked responses for relatively longer durations than conventional methods, which typically require transferring data in small batches due to a relatively small size of a buffer of a cochlear implant.

At operation 706, system 500 may associate the first evoked response data and the second evoked response data with metadata representative of one or more attributes of the stimulation. The one or more attributes of the stimulation may include an amplitude of the stimulation, a starting and/or stopping time of the stimulation, a duration that the stimulation is applied to the recipient, a type of the stimulation (e.g., whether the stimulation is electrical stimulation or acoustic stimulation), and/or any other property of the stimulation as may serve a particular implementation. In this manner, data descriptive of the stimulation may be temporally correlated with the first evoked response data and the second evoked response data.

System 500 may associate the first evoked response data and the second evoked response data with the metadata representative of one or more attributes of the stimulation in any suitable manner. For example, system 500 may include the metadata in one or more files representative of the first and second evoked response data, tag the first and second evoked response data with the metadata, or otherwise link the first evoked response data and the second evoked response data with the metadata in software as may serve a particular implementation.

System 500 may be implemented by any of the components described herein. For example, system 500 may be implemented by a sound processor communicatively coupled to the first cochlear implant by way of a wireless link.

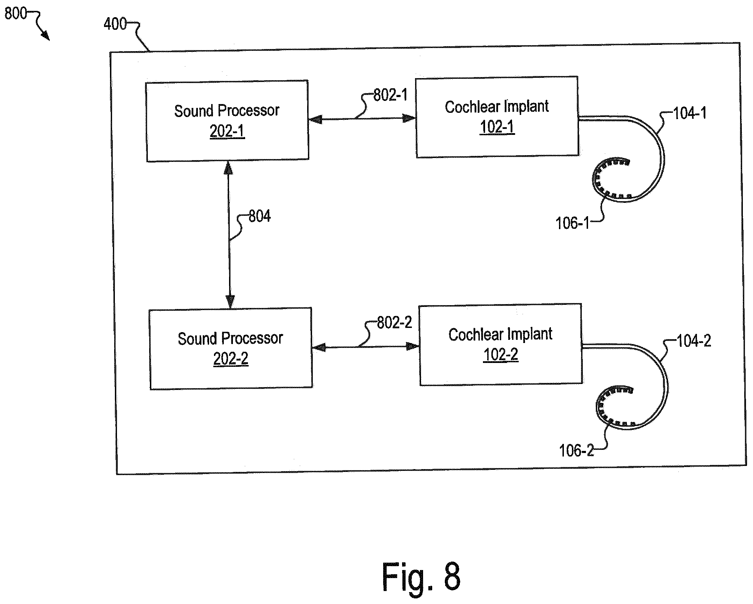

To illustrate, Fig. 8 shows an example configuration 800 in which processing unit 108-1 of bilateral hearing system 400 is implemented by a first sound processor 202-1 communicatively coupled to cochlear implant 102-1 by way of a first wireless link 802-1 and processing unit 108-2 of bilateral hearing system 400 is implemented by a second sound processor 202-2 communicatively coupled to cochlear implant 202-2 by way of a second wireless link 802-2. Sound processors 202-1 and 202-2 may be similar to sound processor 202 described herein. Wireless links 802-1 and 802-2 may be similar to wireless communication link 210. In configuration 800, system 500 is implemented by sound processor 202-1.

As shown, sound processor 202-1 is communicatively coupled by way of a communication link 804 with sound processor 202-2. Communication link 804 may be wired or wireless as may serve a particular implementation. In this configuration, sound processor 202-1 may receive the first evoked response data directly from cochlear implant 102-1 by way of wireless link 802-1 and the second evoked response data from sound processor 202-2 by way of communication link 804.

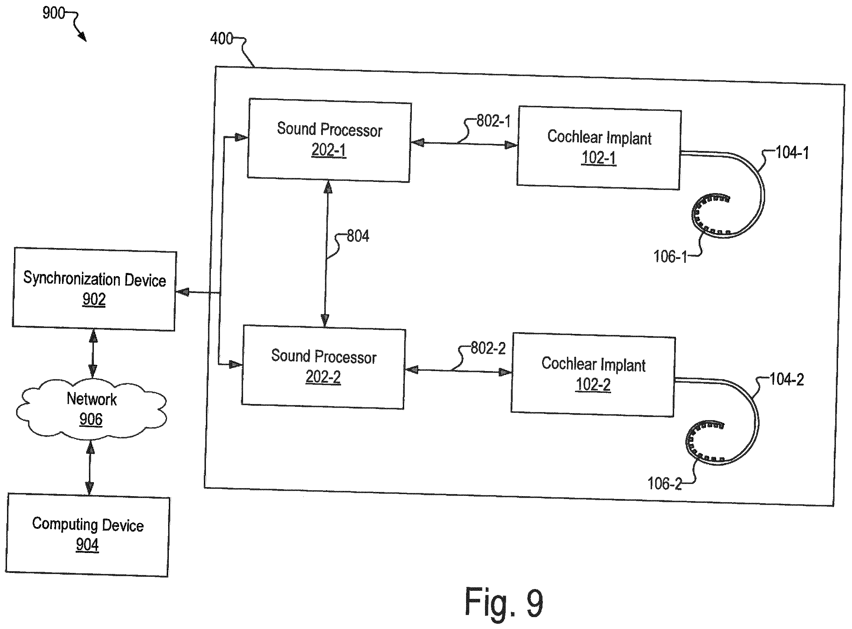

Fig. 9 shows an example configuration 900 in which system 500 is implemented by a synchronization device 902 communicatively coupled to sound processors 202-1 and 202-2. Synchronization device 902 may be implemented by any suitable computing device configured to perform one or more of the operations described herein. Synchronization device 902 may be communicatively coupled to sound processors 202-1 and 202-2 by way of any suitable wired or wireless communication link as may serve a particular implementation.

As shown, in some examples, synchronization device 902 may be communicatively coupled with a computing device 904 by way of a network 906. In this configuration, synchronization device 902 may send (e.g., stream) the first evoked response data and the second evoked response data to computing device 904. Computing device 904 may store and/or otherwise process the first and second evoked response data as may serve a particular implementation. For example, computing device 904 may perform various operations based on the first and second evoked response data.

Network 906 may any suitable wired or wireless network (e.g., the Internet, an intranet, etc.). In alternative examples, synchronization device 902 may be directly connected to computing device 904, obviating the need for network 906.

In other alternative examples, configuration 900 does not include synchronization device 902 or network 906. In these alternative configurations, computing device 904 may be communicatively coupled directly with sound processors 202-1 and 202-2 and configured to perform the operations described herein.

In some examples, system 500 may additionally or alternatively direct cochlear implants 108-1 and 108-2 to record one or more evoked responses in the absence of stimulation. In this manner, system 500 may monitor the brain's resting status (e.g., by monitoring alpha, theta, and/or gamma waves). For example, subsequent to the stimulation being applied and during a time period in which no stimulation is applied to the recipient, system 500 may cause the first cochlear implant and the second cochlear implant to simultaneously record one or more evoked responses within the recipient that occur in the absence of stimulation.

In some examples, a non-transitory computer-readable medium storing computer-readable instructions may be provided in accordance with the principles described herein. The instructions, when executed by a processor of a computing device, may direct the processor and/or computing device to perform one or more operations, including one or more of the operations described herein. Such instructions may be stored and/or transmitted using any of a variety of known computer-readable media.

A non-transitory computer-readable medium as referred to herein may include any non-transitory storage medium that participates in providing data (e.g., instructions) that may be read and/or executed by a computing device (e.g., by a processor of a computing device). For example, a non-transitory computer-readable medium may include, but is not limited to, any combination of non-volatile storage media and/or volatile storage media. Illustrative non-volatile storage media include, but are not limited to, read-only memory, flash memory, a solid-state drive, a magnetic storage device (e.g., a hard disk, a floppy disk, magnetic tape, etc.), ferroelectric random-access memory (RAM), and an optical disc (e.g., a compact disc, a digital video disc, a Blu-ray disc, etc.). Illustrative volatile storage media include, but are not limited to, RAM (e.g., dynamic RAM).

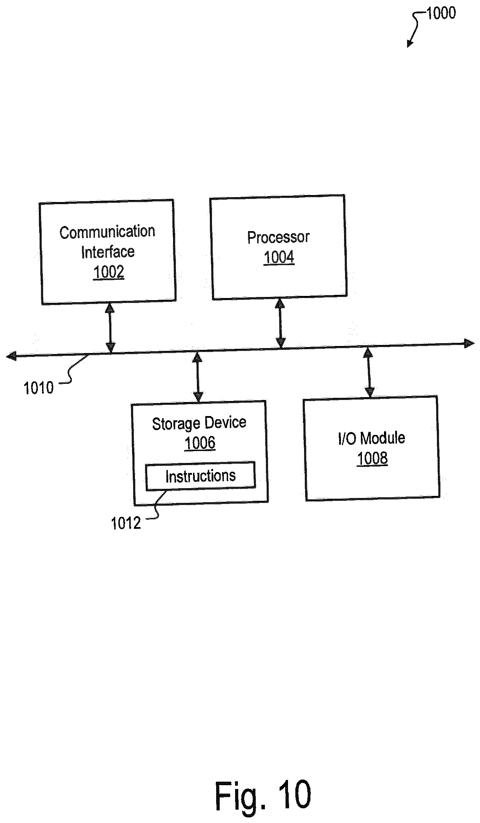

Fig. 10 illustrates an illustrative computing device 1000 that may be specifically configured to perform one or more of the processes described herein. As shown in Fig. 10, computing device 1000 may include a communication interface 1002, a processor 1004, a storage device 1006, and an input/output (I/O) module 1008 communicatively connected one to another via a communication infrastructure 1010. While an illustrative computing device 1000 is shown in Fig. 10, the components illustrated in Fig. 10 are not intended to be limiting. Additional or alternative components may be used in other embodiments. Components of computing device 1000 shown in Fig. 10 will now be described in additional detail.

Communication interface 1002 may be configured to communicate with one or more computing devices. Examples of communication interface 1002 include, without limitation, a wired network interface (such as a network interface card), a wireless network interface (such as a wireless network interface card), a modem, an audio/video connection, and any other suitable interface.

Processor 1004 generally represents any type or form of processing unit capable of processing data and/or interpreting, executing, and/or directing execution of one or more of the instructions, processes, and/or operations described herein. Processor 1004 may perform operations by executing computer-executable instructions 1012 (e.g., an application, software, code, and/or other executable data instance) stored in storage device 1006.

Storage device 1006 may include one or more data storage media, devices, or configurations and may employ any type, form, and combination of data storage media and/or device. For example, storage device 1006 may include, but is not limited to, any combination of the non-volatile media and/or volatile media described herein. Electronic data, including data described herein, may be temporarily and/or permanently stored in storage device 1006. For example, data representative of computer-executable instructions 1012 configured to direct processor 1004 to perform any of the operations described herein may be stored within storage device 1006. In some examples, data may be arranged in one or more databases residing within storage device 1006.

I/O module 1008 may include one or more I/O modules configured to receive user input and provide user output. One or more I/O modules may be used to receive input for a virtual experience. I/O module 1008 may include any hardware, firmware, software, or combination thereof supportive of input and output capabilities. For example, I/O module 1008 may include hardware and/or software for capturing user input, including, but not limited to, a keyboard or keypad, a touchscreen component (e.g., touchscreen display), a receiver (e.g., an RF or infrared receiver), motion sensors, and/or one or more input buttons.

I/O module 1008 may include one or more devices for presenting output to a user, including, but not limited to, a graphics engine, a display (e.g., a display screen), one or more output drivers (e.g., display drivers), one or more audio speakers, and one or more audio drivers. In certain embodiments, I/O module 1008 is configured to provide graphical data to a display for presentation to a user. The graphical data may be representative of one or more graphical user interfaces and/or any other graphical content as may serve a particular implementation.

Advantages and features of the present disclosure can be further described by the following statements:
1. A system comprising: a memory storing instructions; and one or more processors communicatively coupled to the memory and configured to execute the instructions to perform a process comprising: directing stimulation to be applied to a recipient of a bilateral hearing system that includes a first cochlear implant associated with a first ear of the recipient and a second cochlear implant associated with a second ear of the recipient; and causing the first cochlear implant and the second cochlear implant to simultaneously record, during an acquisition time window, one or more evoked responses within the recipient that are elicited by the stimulation.
2. The system of statement 1, wherein the process further comprises: receiving, from the first cochlear implant by way of a first back telemetry channel, first evoked response data representative of the one or more evoked responses recorded by the first cochlear implant; receiving, from the second cochlear implant by way of a second back telemetry channel, second evoked response data representative of the one or more evoked responses recorded by the second cochlear implant; and associating the first evoked response data and the second evoked response data with metadata representative of one or more attributes of the stimulation.
3. The system of any of the preceding statements, wherein the one or more attributes of the stimulation comprise at least one of an amplitude of the stimulation, a start time of the stimulation, a stop time of the stimulation, a duration that the stimulation is applied to the recipient, or a type of the stimulation.
4. The system of any of the preceding statements, wherein the system is implemented by a sound processor communicatively coupled to the first cochlear implant by way of a wireless link.
5. The system of any of the preceding statements, wherein: the sound processor is communicatively coupled by way of a communication link with an additional sound processor that is communicatively coupled to the second cochlear implant by way of an additional wireless link; and the sound processor receives the second evoked response data by way of the additional sound processor and the communication link.
6. The system of any of the preceding statements, wherein the system is implemented by a synchronization device communicatively coupled to: a first sound processor communicatively coupled to the first cochlear implant by way of a first wireless link; and a second sound processor communicatively coupled to the second cochlear implant by way of a second wireless link.
7. The system of any of the preceding statements, wherein the process further comprises the synchronization device sending the first evoked response data and the second evoked response data to a computing device.
8. The system of any of the preceding statements, wherein the directing the stimulation to be applied to the recipient comprises directing the first cochlear implant to apply electrical stimulation to the recipient by way of at least one electrode electrically coupled to the first cochlear implant.
9. The system of any of the preceding statements, wherein the directing the stimulation to be applied to the recipient further comprises directing the second cochlear implant to apply additional electrical stimulation to the recipient by way of at least one electrode electrically coupled to the second cochlear implant.
10. The system of any of the preceding statements, wherein the directing the stimulation to be applied to the recipient further comprises applying acoustic stimulation to the recipient.
11. The system of any of the preceding statements, wherein the stimulation comprises at least one of electrical stimulation or acoustic stimulation.
12. The system of any of the preceding statements, wherein the first cochlear implant is configured to record the one or more evoked responses using at least one electrode electrically connected to the first cochlear implant.
13. The system of any of the preceding statements, wherein the causing the first cochlear implant and the second cochlear implant to simultaneously record, during the acquisition time window, the one or more evoked responses comprises: transmitting a first command to the first cochlear implant and the second cochlear implant that causes the first cochlear implant and the second cochlear implant to start recording the one or more evoked responses at a first point in time that defines a beginning of the acquisition time window.
14. The system of any of the preceding statements, wherein the causing the first cochlear implant and the second cochlear implant to simultaneously record, during the acquisition time window, the one or more evoked responses further comprises transmitting a second command to the first cochlear implant and the second cochlear implant that causes the first cochlear implant and the second cochlear implant to stop recording the one or more evoked responses at a second point in time that defines an end of the acquisition time window.
15. The system of any of the preceding statements, wherein the process further comprises: causing, subsequent to the stimulation being applied and during a time period in which no stimulation is applied to the recipient, the first cochlear implant and the second cochlear implant to simultaneously record one or more additional evoked responses within the recipient that occur in the absence of stimulation.
16. A system comprising: a first cochlear implant associated with a first ear of a recipient and electrically coupled to a first electrode; a second cochlear implant associated with a second ear of the recipient and electrically coupled to a second electrode; and a computing device communicatively coupled with the first cochlear implant and with the second cochlear implant and configured to perform a process comprising: directing stimulation to be applied to the recipient, and causing the first cochlear implant and the second cochlear implant to simultaneously record, during an acquisition time window and using the first and second electrodes, one or more evoked responses within the recipient that are elicited by the stimulation.
17. The system of any of the preceding statements, wherein the process further comprises: receiving, from the first cochlear implant by way of a first back telemetry channel, first evoked response data representative of the one or more evoked responses recorded by the first cochlear implant; receiving, from the second cochlear implant by way of a second back telemetry channel, second evoked response data representative of the one or more evoked responses recorded by the second cochlear implant; and associating the first evoked response data and the second evoked response data with metadata representative of one or more attributes of the stimulation.
18. The system of any of the preceding statements, wherein the causing the first cochlear implant and the second cochlear implant to simultaneously record, during the acquisition time window, the one or more evoked responses comprises: transmitting a first command to the first cochlear implant and the second cochlear implant that causes the first cochlear implant and the second cochlear implant to start recording the one or more evoked responses at a first point in time that defines a beginning of the acquisition time window.
19. The system of any of the preceding statements, wherein the causing the first cochlear implant and the second cochlear implant to simultaneously record, during the acquisition time window, the one or more evoked responses further comprises transmitting a second command to the first cochlear implant and the second cochlear implant that causes the first cochlear implant and the second cochlear implant to stop recording the one or more evoked responses at a second point in time that defines an end of the acquisition time window.
20. A method comprising: directing stimulation to be applied to a recipient of a bilateral hearing system that includes a first cochlear implant associated with a first ear of the recipient and a second cochlear implant associated with a second ear of the recipient; and causing the first cochlear implant and the second cochlear implant to simultaneously record, during an acquisition time window, one or more evoked responses within the recipient that are elicited by the stimulation.

In the preceding description, various illustrative embodiments have been described with reference to the accompanying drawings. It will, however, be evident that various modifications and changes may be made thereto, and additional embodiments may be implemented, without departing from the scope of the invention as set forth in the claims that follow. For example, certain features of one embodiment described herein may be combined with or substituted for features of another embodiment described herein. The description and drawings are accordingly to be regarded in an illustrative rather than a restrictive sense.

## Claims

1. A system comprising:
a memory storing instructions; and
one or more processors communicatively coupled to the memory and configured to execute the instructions to perform a process comprising:
directing stimulation to be applied to a recipient of a bilateral hearing system that includes a first cochlear implant associated with a first ear of the recipient and a second cochlear implant associated with a second ear of the recipient; and
causing the first cochlear implant and the second cochlear implant to simultaneously record, during an acquisition time window, one or more evoked responses within the recipient that are elicited by the stimulation.

2. The system of claim 1, wherein the process further comprises:
receiving, from the first cochlear implant by way of a first back telemetry channel, first evoked response data representative of the one or more evoked responses recorded by the first cochlear implant;
receiving, from the second cochlear implant by way of a second back telemetry channel, second evoked response data representative of the one or more evoked responses recorded by the second cochlear implant; and
associating the first evoked response data and the second evoked response data with metadata representative of one or more attributes of the stimulation.

3. The system of claim 2, wherein the one or more attributes of the stimulation comprise at least one of an amplitude of the stimulation, a start time of the stimulation, a stop time of the stimulation, a duration that the stimulation is applied to the recipient, or a type of the stimulation.

4. The system of any one of claims 2-3, wherein the system is implemented by a sound processor communicatively coupled to the first cochlear implant by way of a wireless link.

5. The system of any one of claims 2-4, wherein:
the sound processor is communicatively coupled by way of a communication link with an additional sound processor that is communicatively coupled to the second cochlear implant by way of an additional wireless link; and
the sound processor receives the second evoked response data by way of the additional sound processor and the communication link.

6. The system of claim 2, wherein the system is implemented by a synchronization device communicatively coupled to:
a first sound processor communicatively coupled to the first cochlear implant by way of a first wireless link; and
a second sound processor communicatively coupled to the second cochlear implant by way of a second wireless link.

7. The system of claim 6, wherein the process further comprises the synchronization device sending the first evoked response data and the second evoked response data to a computing device.

8. The system of any of the preceding claims, wherein the directing the stimulation to be applied to the recipient comprises directing the first cochlear implant to apply electrical stimulation to the recipient by way of at least one electrode electrically coupled to the first cochlear implant.

9. The system of claim 8, wherein the directing the stimulation to be applied to the recipient further comprises directing the second cochlear implant to apply additional electrical stimulation to the recipient by way of at least one electrode electrically coupled to the second cochlear implant.

10. The system of claim 8, wherein the directing the stimulation to be applied to the recipient further comprises applying acoustic stimulation to the recipient.

11. The system of any of the preceding claims, wherein the first cochlear implant is configured to record the one or more evoked responses using at least one electrode electrically connected to the first cochlear implant.

12. The system of any of the preceding claims, wherein the causing the first cochlear implant and the second cochlear implant to simultaneously record, during the acquisition time window, the one or more evoked responses comprises:
transmitting a first command to the first cochlear implant and the second cochlear implant that causes the first cochlear implant and the second cochlear implant to start recording the one or more evoked responses at a first point in time that defines a beginning of the acquisition time window.

13. The system of claim 12, wherein the causing the first cochlear implant and the second cochlear implant to simultaneously record, during the acquisition time window, the one or more evoked responses further comprises transmitting a second command to the first cochlear implant and the second cochlear implant that causes the first cochlear implant and the second cochlear implant to stop recording the one or more evoked responses at a second point in time that defines an end of the acquisition time window.

14. The system of any of the preceding claims, wherein the process further comprises:
causing, subsequent to the stimulation being applied and during a time period in which no stimulation is applied to the recipient, the first cochlear implant and the second cochlear implant to simultaneously record one or more additional evoked responses within the recipient that occur in the absence of stimulation.

15. A system comprising:
a first cochlear implant associated with a first ear of a recipient and electrically coupled to a first electrode;
a second cochlear implant associated with a second ear of the recipient and electrically coupled to a second electrode; and
a computing device communicatively coupled with the first cochlear implant and with the second cochlear implant and configured to perform a process comprising:
directing stimulation to be applied to the recipient, and
causing the first cochlear implant and the second cochlear implant to simultaneously record, during an acquisition time window and using the first and second electrodes, one or more evoked responses within the recipient that are elicited by the stimulation.
